Europäisches Patentamt

European Patent Office

Office européen des brevets

(11) Publication number: **0 407 816 A2**

## EUROPEAN PATENT APPLICATION

(21) Application number: 90112260.6

(22) Date of filing: 27.06.90

(51) Int. Cl.⁵: **C07H 19/073**, C07H 19/10, C07H 21/04

(30) Priority: 14.07.89 US 380569

(43) Date of publication of application:
16.01.91 Bulletin 91/03

(84) Designated Contracting States:
DE ES FR IT

(71) Applicant: **ABBOTT LABORATORIES**
**CHAD-0377, AP6D/2, One Abbott Park Road**
**Abbott Park, Illinois 60064-3500(US)**

(72) Inventor: **Celebuski, Joseph E.**
**5080 Fox Lane**
**Gurnee, Illinois 60031(US)**

(74) Representative: **Modiano, Guido et al**
**MODIANO, JOSIF, PISANTY & STAUB**
**Modiano & Associati Via Meravigli, 16 16 16**
**I-20123 Milano(IT)**

(54) **Base modified nucleosides.**

(57) The present invention relates to the preparation and isolation of 5-position modified uracil nucleosides and nucleotides and to their use in the synthesis of labeled oligonucleotides useful as nucleic acid probes. More specifically, the present invention provides for improved methods for the preparation of novel 5-aza uracil nucleoside derivatives wherein a 5-halo-uracil nucleoside is reacted with a bifunctional amine, such as diamines, a sulfhydrylamino compound, or an hydroxyamino compounds, to yield the corresponding 5-(aza-aminoalkyl)-uracil nucleoside, 5-(aza-sulfhydrylalkyl)uracil nucleoside or 5-(aza-hydroxyalkyl)uracil nucleoside, respectively, and for improved methods for the isolation of the products formed. These uracil nucleoside derivatives can be used as substrates in a method for preparing novel labeled oligonucleotides of DNA. Reporter groups can be attached either before or after the preparation of the oligonuclectides. These reporter group labeled oligonucleotides are especially useful when incorporated into nucleic acid probes which readily can be used in hybridization studies to form hybrids with either DNA or RNA.

Fig 1

EP 0 407 816 A2

# BASE MODIFIED NUCLEOSIDES

## BACKGROUND OF THE INVENTION

### 1. Technical Field

The present invention relates to the preparation and isolation of 5-position modified uracil nucleosides and nucleotides and their use for synthesis of labeled oligonucleotides.

### 2. Description of Related Art

Processes for labeling nucleic acids have found significant utility in many procedures used in biomedical and recombinant DNA research. Matthews, J.A., et al ., Anal. Biochem. , 169:1 (1988). Derivatized and/or labeled bases, nucleosides and nucleotide analogs can be used to make indicator probes useful for detecting, monitoring, localizing and/or isolating nucleic acids. Important considerations in the preparation of nucleic acid probes include whether the level of incorporation of a derivatized or labeled substance will be sufficiently high to allow ready detection of the probe, whether the substance is readily recognized as a substrate by required polymerases during probe manufacture, and/or whether the corresponding phosphoramidite derivatives can be used in automated oligonucleotide synthesis systems such as those described in Van Brunt, J., Bio/Tech , 3:775 (1985) and in Haralambidis, J., et al ., Nucleic Acids Res. , 15:4857 (1987).

Although a variety of methods have been described to obtain chemically modified bases. nucleosides, and nucleotides, some of these methods are not amenable for use in the preparation of labeled nucleic acid probes. See for example, Friedland, M., et al ., Biochem. Biophys. Acta. , 51:148 (1961); Trayer, I.P., et al ., Biochem. J. , 139:609 (1974); Dale, R.M.K., et al ., Biochem. , 14:2447 (1975). A number of preparative methods have resulted in derivatives having a site of unsaturation immediately adjacent to the pyrimidine nucleus. These methods therefore require another step to reduce the site of unsaturation. See for example, Bergstrom, D.E., et al ., J. Am. Chem. Soc. , 98:1587 (1976); Bergstrom, D.E., et al ., J. Am. Chem. Soc. , 100:26 (1978); Bergstorm, D.E., J. Org. Chem. , 46:1432 (1981); and, Robins, M.J., et al ., J. Org. Chem. , 48:1854 (1983). See also, Langer, P.R., et al ., P.N.A.S. , 78:6633 (1971); Saito, I., J. Org. Chem. , 51:5148 (1986); and, Hampton, A., et al ., J. Med. Chem. , 22:621 (1979).

Ruth and co-workers [Jablonski, E., et al ., Nucleic Acids Res. , 14:6115-6128 (1986); Ruth, J.L., DNA , 3:123 (1986); Ruth, J.L., et al ., DNA , 4:93 (1985); and Jablonski, E., et al ., DNA , 5:89 (1986)] have reported C-5 substituted deoxyuridine compounds which can be incorporated internally into an oligonucleotide. For example, substituted uracil and cytosine nucleoside analogs, with a linker-arm of from four to nineteen atoms in length attached at C-5, were described in Ruth. J.L., 4th Annual Congress for Recombinant DNA Research, DNA , 5:123 (1986). The linker arms terminate in chemically reactive functionalities (primary amines. carboxylic acid) not normally found in DNA. These derivatives, were prepared by reacting methyl acrylate with 5- chloromercuri-2'-deoxyuridine under $Li_2PdCl_4$ catalysis to form 5-(methyl acrylyl)-2'-deoxyuridine which is then hydrolyzed. The resultant acid is then reacted with a diamine. The oligonucleotides obtained reportedly kinased normally and were hybridized to target plasmid DNA with no discernible difference relative to unmodified probes. Oligomers further modified by the attachment of non radioactive reporter groups such as fluorescein, dinitrobenzene, or biotin, still kinased normally and exhibited little if any change in hybridization selectivity or strength.

Jablonski, E., et al ., DNA , 5:89 (1986) described a method requiring a minimum of three steps for the linkage, to alkaline phosphatase and several other enzymes, of 5-substituted, amine-terminated thymidines in oligonucleotides.

Despite these known methods, there continues to exist a need in the art for a method for preparing a variety of modified bases, nucleosides and nucleotides which are stable to mild electrophiles and nucleophiles, which are relatively simple and cost-efficient to prepare, which require a minimum of synthetic steps to prepare, which retain their biological activity as substrates for RNA and/or DNA polymerase and which will give multiple labeling upon incorporation into oligonucleotides.

## BRIEF DESCRIPTION OF THE DRAWINGS

Figure 1 shows the structure of 5-(1-aza-7-aminoheptyl)-2'-deoxyuridine;

Figure 2 shows the structure of 5-(1-aza-7-aminoheptyl)-2'-deoxyuridine-5'-triphosohate;

Figure 3 shows the structure of 5-(1-aza-5,10-dioxa-13-aminotridecyl)-2'-deoxyuridine;

Figure 4 shows the structure of 5-(-aza-5,10 dioxa-13-aminotridecyl)-2'-deoxyuridine-5'-triphosphate;

Figure 5 shows the structure of 3-(4-[N-(3-sulfopropyl)-N-[N'-(3-sulfopropyl)acridin-9-ylcarbonyl]-sulfonamido]phenyl)propionic acid (SSC acridine);

Figure 6 shows the structure of 3-(4-[N-(3-isopropyl)-N-[N'-(3-methyl)acridin-9-ylcarbonyl]sulfonamido]-phenyl)propionic acid (MIC acridine);

Figure 7 shows the structure of 3-(4-[N-(3-isopropyl)-N-[N'-(3-sulfopropyl)acridin-9-ylcarbonyl]-sulfonamido]phenyl)propionic acid (SIC acridine); and

Figure 8 shows the structure of 5-(1-aza-7[3-(4-[N-(3-sulfopropyl)-N-[N'-(3-sulfopropyl-)acridin-9-ylcarbonyl]sulfonamido]phenyl)propionamido]heptyl)-2'-deoxyuridine-5'-triphosphate.

## BRIEF SUMMARY OF THE INVENTION

The present invention relates to the preparation and isolation of 5-position modified uracil nucleosides and nucleotides and to their use in the synthesis of labeled oligonucleotides useful as nucleic acid probes. More specifically, the present invention provides for improved methods for the preparation of novel 5-aza uracil nucleoside derivatives wherein a 5-halo-uracil nucleoside is reacted with a bifunctional amine, such as diamine, a sulfhydrylamino compound, or a hydroxyamino compound, to yield the corresponding 5-(aza-aminoalkyl)-uracil nucleoside, 5-(aza- sulfhydrylalkyl)-uracil nucleoside or 5-(aza-hydroxyalkyl)-uracil nucleoside, respectively, more generally referred to as terminally reactive aza-alkyl uracil nucleosides. The present invention also provides for improved methods for the isolation of the products formed.

According to these improved methods, the 5-halo-uracil nucleoside can be 5-iodo-uracil nucleoside, 5-bromo-uracil nucleoside, or 5-chloro-uracil nucleoside. The bifunctional amine can be a straight or branched chain, and is of the general formula:

$$H_2N-(CH_2)_u[(CH_2)_vO(CH_2)_w]_x[(CH_2)_vO(CH_2)_w]_y(CH_2)_z-T$$

wherein:

u is 1 to 20,

v is 1 to 5,

w is 1 to 5,

x is 0 to 1,

y is 0 to 5,

z is 0 to 20; and

the sum of u, v, w, x, y, and z is 5 to 20;

T is $-CH_2A$, $-CH-((CH_2)_nA)_2$, or $-C((CH_2)_nA)_3$;

n is 1 to 4; and

A is $NH_2$, SH, or OH;

Preferably, the bifunctional amine is of the formula:

$$NH_2(CH_2)_3-O-(CH_2)_4-O-(CH_2)_3-NH_2,$$

and $NH_2(CH_2)_6NH_2$.

Methods are also provided by the present invention wherein the terminally reactive aza-alkyl uracil nucleosides are further modified through the addition of reporter groups attached via a condensation reaction with the free amino. sulfhydryl, or hydroxyl group at the 5-position of the pyrimidine ring. In general, the reporter group can be any label capable of providing a detectable signal. The label can be any of the various types of signal producing species.

These uracil nucleoside derivatives can be used as substrates in methods, including automated methods, for preparing labeled oligonucleotides of DNA. Briefly, both a template of the DNA molecule to be copied and a primer are provided, the primer is annealed to the template, four substrates, namely, the four deoxynucleoside 5'-triphosphates (dNTP), dATP, dGTP, dCTP and dTTP, are added, and the primer is extended using DNA polymerase to form the oligonucleotides. The uracil nucleoside derivatives of the invention can be advantageously substituted for dTTP.

In the automated method for preparing oligonucleotides, a largely conventional methodology of solid phase phosphoramidite chemistry (Beaucage & Caruthers, Tetrahedron Letters , 22(20):1859-1862 (1981)

EP 0 407 816 A2

can be utilized. Briefly, four substrates, namely dA (deoxyadenosine), dG (deoxyguanosine), dC (deoxycytidine) and dT (thymidine) are suitably protected; the 5′-dimethoxytrityl 3′-beta-cyanoethylphosphoramidite derivatives of the four substrates are synthesized; the derivative of one of the four substrates, corresponding to a particular base of the desired DNA sequence of the oligonucleotide, is added to a solid phase bound DNA molecule by dissolving the derivative in an anhydrous solvent; the resultant adduct is washed with organic acid; and the next nucleoside derivative is added. The uracil nucleoside derivatives of the invention can be advantageously substituted for dT.

In both the manual and automated method for preparing labeled oligonucleotides, the reporter groups can be attached either before or after the preparation of the oligonucleotides. The reporter groups are attached by condensing the reactive terminus of the terminally reactive aza-alkyl uracil nucleoside derivatives with the reporter groups. These reporter group labeled oligonucleotides are especially useful when incorporated into nucleic acid probes which readily can be used in hybridization studies to form hybrids with either DNA or RNA.

Also provided by the invention are novel compounds wherein the 5-position of the pyrimidine base nucleus is substituted with a bifunctional amine. Specifically, these uracil nucleoside derivatives are of the formula:

P-S-U-N-R

wherein:

P is a member selected from the group consisting of hydroxyl, monophosphate, diphosphate and triphosphate;

S is a member selected from the group consisting of furanoses, pyranoses, cyclopentane derivatives, cyclohexane derivatives and straight or branched chain alkane derivatives of from 2 to 6 carbon atoms;

U is uracil; and

R is of the formula:

$-(CH_2)_u[(CH_2)_vO(CH_2)_w]_x[(CH_2)_vO(CH_2)_w]_y(CH_2)_z\text{-}T\text{-}B$

wherein:

u is 1 to 20,

v is 1 to 5,

w is 1 to 5,

x is 0 to 1,

y is 0 to 5,

z is 0 to 20; and

the sum of u, v, w, x, y, and z is 5 to 20;

T is $-CH_2A$, $-CH\text{-}[(CH_2)_nA]_2$, or $-C\text{-}[(CH_2)_nA]_3$;

n is 1 to 4;

A is NH, S, or O; and

B is H or a reporter group.

Novel reporter group-labeled oligonucleotides are also presented by the present invention including oligonucleotides prepared by polymerization of reporter group labeled nucleotide triphosphates, as well as ex post facto labeling of oligonucleotides with reporter groups. The compounds of the invention also have potential uses as nucleic acid probes, as in-vivo bacterial enzyme inhibitors and as anti-viral agents. For example, the compounds may be administered as uridine analogs in an amount effective to treat bacterial or viral infections.

Other aspects and advantages of the present invention will be apparent upon consideration of the following detailed description thereof which includes numerous illustrative examples of the practice of the invention.


## DETAILED DESCRIPTION OF THE INVENTION


The present invention includes a method for preparing a uracil nucleoside derivative of the formula:

S-U-NH-R

wherein S is a member selected from the group consisting of furanoses, pyranoses, cyclopentane derivatives, cyclohexane derivatives and straight or branched chain alkane derivatives of from 2 to 6 carbon atoms; U is uracil; and R is of the formula:

$-(CH_2)_u[(CH_2)_vO(CH_2)_w]_x[(CH_2)_vO(CH_2)_w]_y(CH_2)_z\text{-}T$

wherein:

4

u is 1 to 20,

v is 1 to 5,

w is 1 to 5,

x is 0 to 1,

y is 0 to 5,

z is 0 to 20; and

the sum of u, v, w, x, y, and z is 5 to 20;

T is $-CH_2A$, $-CH-[(CH_2)_nA]_2$, or $-C-[(CH_2)_nA]_3$;

n is 1 to 4; and

A is $NH_2$, SH, or OH;

comprising the steps of:

a) reacting a 5 halo uracil nucleoside with a bifunctional amine of the formula:

$H_2N-(CH_2)_u[(CH_2)_vO(CH_2)_w]_x[(CH_2)_vO(CH_2)_w]_y(CH_2)_z-T$

wherein:

u is 1 to 20,

v is 1 to 5,

w is 1 to 5,

x is 0 to 1,

y is 0 to 5,

z is 0 to 20; and the sum of u, v, w, x, y, and z is 5 to 20;

T is $-CH_2A$, $-CH-[(CH_2)_nA]_2$, or $-C-[(CH_2)_nA]_3$;

n is 1 to 4; and

A is $NH_2$, SH, or OH whereby a reaction mixture is formed containing a 5-aza uracil nucleoside derivative; and

b) isolating from said reaction mixture said 5-aza uracil nucleoside derivative.

A reporter group, or signal generating label, may be optionally attached to the free amino, sulfhydryl, or hydroxyl group at the 5 position of the uracil base. The number of atoms in the carbon chain of the bifunctional amine generally depends on the particular reporter group attached. The amine chain must be long enough to avoid interference between the reporter group and the nucleic acid to be hybridized. The maximal values for u, v, w, x, y, z and n are determined by solubility considerations, namely as the sum of u + z increases, the chain becomes more waxy and less water soluble and is therefore less useful in the present invention. To ensure adequate water solubility and fluidity of the chain, the ratio of the number of $-OCH_2$-groups to the number of $-CH_2-$ groups, when the sum of u and z is 7 or greater, should preferably be 1 to 5 such that the chain is hydrophilic and wherein the sum of u, v, w, x, y, z, and n is such that the chain length of the molecule is from about 11 to about 20 Angstroms.

It is envisioned that various bifunctional amines other than the diamines described can also be utilized in the present invention. Although the preferred bifunctional amines are linear terminal diamines, such as 1,6-diamine hexane and 4,9-dioxa-1,12-diaminododecane and heterobifunctional amines, such as 6-amino-1-hexanol and 6-amino-1-hexanethiol, it is not intended to preclude polyfunctional amines, such as tris-(2-aminoethyl) amines, or branched chain polyamines, such as bis-(2-aminoethyl)-N-(ethanethiol)-amine, or tris-N',N',N'-trimethyl-(2-amino ethyl)-amine, and the like, or any other bifunctional amines, from being included in the scope of the present invention.

Various uracil nucleosides, represented as "S-U" in the formula "S-U-NH-R" above, other than uridine and deoxyuridine, can also be utilized in the present invention. Forms of sugars useful in the present invention include the five-membered ring furanoses and the six-membered ring pyranoses. Although the preferred nucleosides are uridine and deoxyuridine, other nucleosides such as 2',3'- dideoxyuridine, arabinosyluracil and 2'-deoxyarabinosyluracil are useful according to the invention. In addition, it is not intended to preclude other ring structures known to those skilled in the art, such as N-1-(hydroxy cyclopentyl) uracil, or heterosubstituted rings, as well as open chain sugar derivatives, such as N-1-$(CH_2OCH_2CH_2OH)$ uracil, from being included within the scope of the present invention. It is further contemplated that the corresponding nucleotides, including monophosphates, diphosphates and triphosphates of the above-mentioned uracil nucleosides, can also be utilized in the present invention.

The present invention also provides for improved methods for isolating these 5-aza uracil nucleoside derivatives wherein the initial products formed from the reaction of the base or nucleoside with the bifunctional amine are applied to a sized, analytical grade, cation exchange column, for example AG 50W-X8 ($NH_4$ form) [BioRad Laboratories, Richmond, CA], a sulfonic acid functional group resin, and the column is eluted with a basic buffer, such as ammonium hydroxide.

In addition, it is contemplated that ion exchange resins, which vary in the amount of cross-linking, can also be utilized to isolate the 5-aza uracil nucleosides of the invention. For example, resins other than Bio-Rad AG 50W-X8, such as Bio-Rad AG 50W-X4 will be effective in the methods of the invention. In addition, resins with alternate types of counter-ions can also be used in conjunction with the correspondingly appropriate concentration of basic buffer needed to elute the nucleosides. For example, the H form of the resin Bio-Rad AG 50W-X8 can be used instead of the $NH_4$ form. Similarly, basic buffers other than ammonium hydroxide, such as ethylene diamine. can also be used for the elution of the uracil nucleosides of the invention from the ion exchange resin.

The present invention further provides for methods for the phosphorylation of the 5-aza uracil nucleoside compounds wherein a phosphate group is attached to the 5' carbon of the sugar or other ring structure by a phosphoester linkage to provide corresponding monophosphates, diphosphates and triphosphates of the 5-aza uracil nucleosides. Michelson, A.M., The Chemistry of Nucleosides and Nucleotides , Academic Press, New York, 1963.

Additional methods provide where the terminally reactive aza-alkyl uracil nucleosides are further modified through the addition of reporter groups attached via a condensation reaction with the free amino, sulfhydryl, or hydroxyl group at the 5-position of the pyrimidine ring.

The reporter group can be any label capable of providing a detectable signal. In preferred embodiments, these reporter groups include fluorescent compounds, such as fluorescein and rhodamine; chemiluminescent compounds, such as acridines and luminols; non-radioactive isotopes such as europium, and metal chelators such as ethylenediamine tetra acetate and diethylenetriamine penta-acetate, for time-resolved fluorescence; radiometals; enzymes, such as alkaline phosphatase, acid phosphatase, beta-galactosidase. horseradish peroxidase, and the like; and liposomes. Alternatively, the reporter group can be one component of a two-component signal producing system, the other component being attached to a signal producing species. For example, hapten/labeled anti-hapten systems, such as a biotin/labeled anti biotin system wherein the reporter group is biotin, can be used. Other two-component signal producing systems, such as antigen/labeled antibody systems can also be utilized. For example, fluorescein or digoxin can be utilized as the reporter group in combination with labeled anti-fluorescein or anti-digoxin, respectively.

In addition, reporter groups other than those described above can be useful in the present invention.

Also provided by the invention are novel compounds wherein the 5-position of the pyrimidine base nucleus is substituted with a bifunctional amine. Specifically, these uracil nucleoside derivatives are of the formula:

P-S-U-NH-R

wherein:

P is a member selected from the group consisting of hydroxyl, monophosphate, diphosphate and triphosphate;

S is a member selected from the group consisting of furanoses, pyranoses, cyclopentane derivatives, cyclohexane derivatives and straight or branched chain alkane derivatives of from 2 to 6 carbon atoms;

U is uracil; and

R is of the formula:

$-(CH_2)_u[(CH_2)_vO(CH_2)_w]_x[(CH_2)_vO(CH_2)_w]_y(CH_2)_z$-T-B

wherein:

u is 1 to 20,

v is 1 to 5,

w is 1 to 5,

x is 0 to 1,

y is 0 to 5,

z is 0 to 20; and

the sum of u, v, w, x, y, and z is 5 to 20;

T is $-CH_2A$,$-CH-[(CH_2)_nA]_2$, or $-C-[(CH_2)_nA]_3$;

n is 1 to 4;

A is NH, S, or O; and

B is H or a reporter group.

These novel compounds, such as 5-(1-aza-7-aminoheptyl)-2'-deoxyuridine; and 5-(1-aza-5,10-dioxa-13-aminotridecyl)-2'-deoxyuridine; and their respective 5'-triphosphates may be further derivatized by attaching reporter groups, including fluorescent compounds, chemiluminescent compounds, metal chelators, enzymes, biotin and liposomes, to the free amino, hydroxyl, or sulfhydryl group at the 5-position to yield, for example, 5-(1-aza-7-fluoresceinthioureaheptyl)-2'-deoxyuridine-5'-triphosphate; 5-(1-aza-7-[3-(4-[N-(3-sulfopropyl)-N-[N'-(3-sulfopropyl)acridin-9-ylcarbonyl]sulfonamido]phenyl) propionamido]heptyl)-2'-

deoxyuridine-5'-triphosphate; and 5-(1-aza-5,10-dioxa-13-fluoresceinthioureatridecyl)-2'-deoxyuridine-5'-triphosphate. Preferred compounds of the invention include: 5-(1-aza-7-aminoheptyl)-2'-deoxyuridine-5'-triphosphate; 5-(1-aza-7-fluoresceinthioureaheptyl)-2'-deoxyuridine-5'-triphosphate; 5-(1-aza-5,10-dioxa-13-aminotridecyl)-2'-deoxyuridine-5'-triphosphate; and 5-(1-aza-7-[3-(4-[N-(3-sulfopropyl)-N-[N'-(3 sulfopropyl)-acridin-9-ylcarbonyl]sulfonamido]phenyl)propionamido]heptyl)-2'-deoxyuridine-5'-triphosphate.

The following examples illustrate practice of the invention by which the 5-position of a uracil nucleoside can be modified to provide a spacer arm to which reporter groups can be readily attached. Example 1 relates to the modification of deoxyuridine at the 5-position by providing an aminohexylamino spacer arm to form 5-(1-aza-7-aminoheptyl)-2'-deoxyuridine; this compound can be phosphorylated to yield 5-(1-aza-7-aminoheptyl)-2'-deoxyuridine-5'-monophosphate, as in Example 2, or 5-(1-aza-7-aminoheptyl)-2'-deoxyuridine-5'-triphosohate, as in Example 3. In Example 4, the 5-(1-aza-7-aminoheptyl)-2'-deoxyuridine-5'-triphosphate of Example 3 is further derivatized by adding a reporter group, fluorescein isothiocyanate, to the free amino moiety to form the corresponding 5-(1-aza-7-fluoresceinthioureaheptyl)-2'-deoxyuridine-5'-triphosphate. Example 5 relates to yet another modification of deoxy uridine in which the spacer arm diamine is 4,9-dioxa-1,12-diaminododecane and the product formed is 5-(1-aza-5,10 dioxa-13-aminotridecyl)-2'-deoxyuridine; Examples 6 and 7 describe the preparation of the corresponding 5'-monophosphate and 5'-triphosphate derivatives thereof. Example 8 relates to the preparation of chemiluminescent acridine derivatives and their use as reporter groups to further derivatize the nucleoside triphosphate of Example 3 yielding 5-(1-aza-7-[3-(4-[N-(3-sulfopropyl)-N-[N'-(3-sulfopropyl)acridin-9-ylcarbonyl]sulfonamido]phenyl) propionamido]heptyl)-2'-deoxyuridine-5'-triphosphate (the dUTP-SSC acridine adduct). Example 9 relates to the preparation of a DNA oligonucleotide incorporating the dUTP-SSC acridine adduct of Example 8. Example 10 demonstrates the use of the labeled oligonucleotide of Example 9 as a nucleic acid probe.

The examples which follow are for illustrative purposes only and are not intended in any way to limit the scope of the invention.


## EXAMPLE 1


Preparation of 5-(1-aza-7-aminoheptyl)-2'-deoxyuridine:

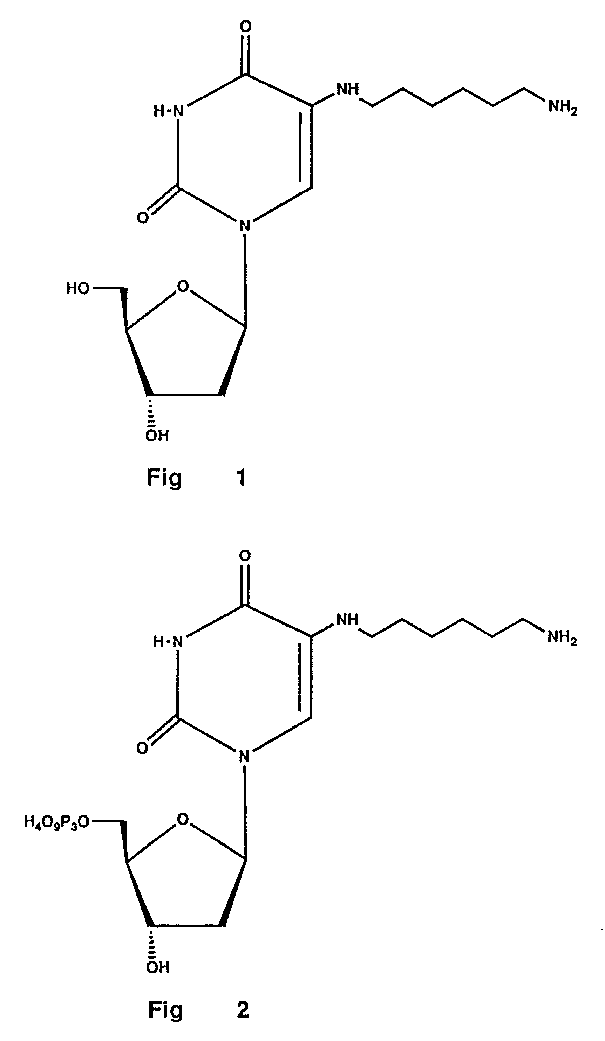

To a solution of 1226.5 mg of 5-iodo-2'-deoxyuridine in 14 mL of DMF was added 2016 mg of 1,6-hexanediamine all at once. The solution was stirred at 50° C for 19 hours. The solvent was stripped off in vacuo , and the residue was thoroughly vacuum dried. The residue was then taken up into water, acidified to pH 2 using 1.5 mL of concentrated HCl, and the resultant solution was then applied to a 2.5 cm ID x 7.5 cm column of AG 50W-X8 ion exchange resin, NH₄ form. The column was eluted first with 125 mL of water; product was then eluted using a step-gradient of 360 mL each of 0.5% and 0.9% ammonium hydroxide. The UV and ninhydrin positive fractions which had an $R_f$ on silica gel of 0.35 in 50:11:30 (v:v:v) n-BuOH:AcOH:H₂O were pooled, stripped to solvent dryness and vacuum dried. Elution of the residue on a 50 cm x 3.5 cm ID column of LH 20 (Pharmacia, Piscataway, NJ) using methanol gave 781.6 mg of 5-(1-aza-7-aminoheptyl)-2'-deoxyuridine as shown in Figure 1, 66% yield.


## EXAMPLE 2


Preparation of 5-(1-aza-7-aminoheptyl)-2'-deoxyuridine-5'-monophosphate:

A 65 mg sample of 5-(1-aza-7-aminoheptyl)-2'- deoxyuridine was taken up in 0.32 mL (EtO)₃PO. The mixture was cooled to 0° C and 35 microliters of POCl₃ and 3 microliters of H₂O was added The reaction mixture was left to stir at 0° C for 2.5 h. 35 microliters more POCl₃ was added, and the reaction mixture was left to stir in a 4° C cold room overnight for a total of 24 h. The nucleotide obtained was precipitated by the addition of 50 mL of Et₂O. The suspension was centrifuged and the supernatant was decanted. The centrifuged pellet was taken up into 10 mL of H₂O and the resulting green solution was heated in a 70° C oil bath for 45 min, and then cooled to room temperature. The solvent was stripped off and the residue was once again dissolved in water and the water again removed. The residue was again taken up with 1 mL of water and this solution was applied to a 5 cm³ column of AG 50W-X8 ion exchange resin made in a disposable 10 cm³ pipette. The column was washed with 30 mL of $H_2O$ and product was eluted with 1% ammonium hydroxide (30 mL). After solvent removal, the product was redissolved in 10 mL of $H_2O$, and 30 mg LiCl was added. Solvent was again removed. LH-20 chromatography (3.5 cm I.D. X 40 cm long column, methanol eluent) gave, upon lyophilization. 42.8 mg of 5- (1-aza-7-aminoheptyl)-2'-deoxyuridine-5'-monophosphate, 53% yield.

EXAMPLE 3

Preparation of 5-( 1-aza-7-aminoheptyl)-2'-deoxyuridine-5'-triphosphate:

To a suspension of 24.9 mg of 5-(1-aza-7-aminoheptyl)-2'-deoxyuridine-5'-monophosphate in 4 mL of MeOH, prepared according to Example 2, was added 24 microliters of NBU₃. The nucleotide was solubilized over the course of 30 minutes. After removal of the MeOH in vacuo , the residue was vacuum dried and then taken up into 8:4 mL dioxane:DMF as much as possible. Then 43 microliters of $CIP(0)(OPh)_2$ and 49 microliters of NBU₃ was added. The reaction mixture was stirred at room temperature for 5 hours, during which time the cloudy suspension became a clear solution. The total volume was reduced to about 0.5 mL and 100 mL of diethyl ether was added to precipitate a sticky gum. The suspension was left in an ice bath for 1 hour and the supernatant was decanted off. The residue was thoroughly vacuum dried and 86 mg of $(NBU_3)_{1.5}H_4P_2O_7$ (Sigma, St. Louis, MO) in 6 mL of pyridine was added. The mixture was left to stir at room temperature for 20 hours. The nucleotide pyridinium salt was precipitated by the addition of 90 mL of $Et_2O$. The precipitate was isolated by centrifugation and taken up into 4:2 mL water:ethanol. The solution was adjusted to pH 4 using 6 drops of 10% HCl, and the acidified solution was stirred at room temperature for 4 hours. The solvents were removed by rotary evaporation, and the residue vacuum dried. LH-20 chromatography (50 cm x 3.5 cm ID, MeOH) gave 19.6 mg of 5-(1-aza-7-aminoheptyl)-2'-deoxyuridine-5'-triphosphate as shown in figure 2, 57% yield.

HPLC purification of the triphosphate was done under the following standard gradient conditions, hereinafter referred to as the standard HPLC protocol.

Flow rate: 1.0 mL/min; Detector: 263 nm; Solvent A: Acetonitrile; Solvent B: 0.1 M $HNEt_3^+$ $OAc^-$; Column: microBondapak C-18 reverse phase (Waters Assocs., Milford, MA).

| Gradient Table: | | |
|---|---|---|
| Time (min) | %A | %B |
| 0 | 5 | 95 |
| 5 | 20 | 80 |
| 35 | 50 | 50 |

Under these conditions, the above-prepared triphosphate had a retention time of 8.3 minutes.

It is also anticipated that modifications of the HPLC purification of the triphosphates of the invention will be similarly effective in the methods of the invention. Although the preferred column material is hydrophobic in nature, such as microBondapak C-18 reverse phase, and the preferred eluants are acetonitrile and 0.1 M triethyl ammonium acetate, it is anticipated that other resins such as size exclusion columns (e.g., Superose 6, Pharmacia, Piscataway, NJ) or ion exchange columns (e.g., Mono Q, Pharmacia), along with the appropriate eluents, e.g., 0.05 M $Na_2HPO_4$/0.15 M NaCl for size exclusion and 20 mM Tris-HCl for ion exchange, will be similarly effective in the methods of the invention.

EXAMPLE 4

Preparation of 5-(1-aza-7-fluoresceinthioureaheptyl)-2′-deoxyuridine-5′-triphosphate:

To a solution of 1.3 mg of 5-(1-aza-7-aminoheptyl)-2′-deoxyuridine-5′-triphosphate in 1.42 mL of pH 9 $Na_2CO_3$ solution, prepared according to Example 3, was added 4.26 mg of fluorescein isothiocyanate. The reaction mixture was occasionally swirled at room temperature over the course of 28 hours. The reaction was quenched using 2 drops of conc. HCl and a precipitate was seen to form. After solvent removal in vacuo , the residue was taken up into 2:1 mL methanol:water. The bright reddish-orange solution was then applied to a 1.5 g LH-20 column made up in the same solvent system. The column was eluted with 2:1 MeOH:$H_2O$ and the slowest-moving fluorescent fraction on TLC (50:11:30 n-BUOH:AcOH:$H_2O$, silica gel) was collected, giving 1.1 mg of 5-(1-aza-7fluoresceinthioureaheptyl)-2′-deoxyuridine-5′-triphosphate, 50% yield. Under the standard HPLC protocol conditions described in Example 3, the product had a retention time of 11.09 minutes.

## EXAMPLE 5

Preparation of 5-(1-aza-5,10-dioxa-13-aminotridecyl)-2′-deoxyuridine:

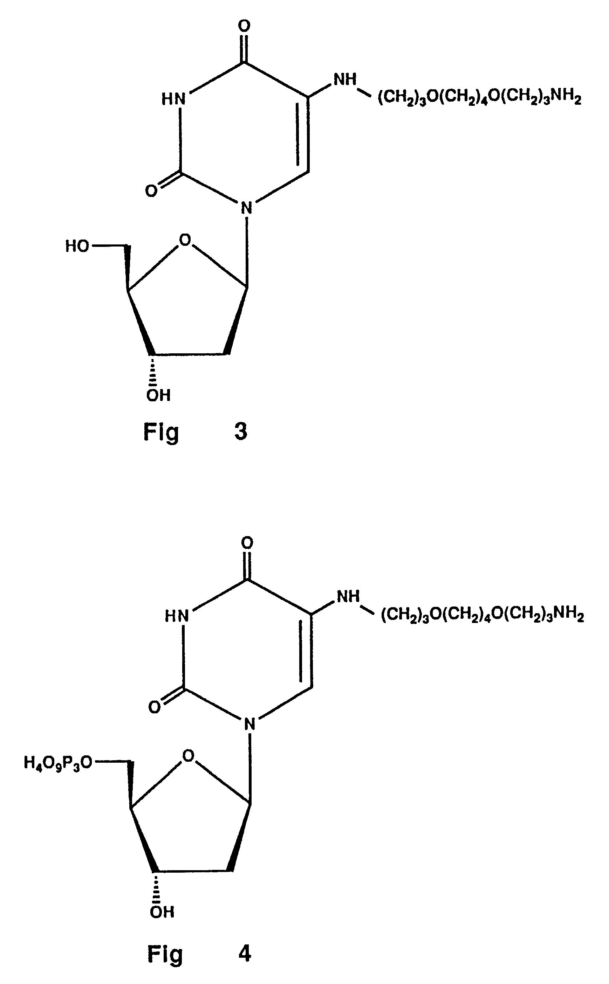

A solution of 2.17 mL of 4,9-dioxa-1, 12-diaminododecane and 906 mg 5-iodo-deoxyuridine in 10 mL of DMF was stirred at 50° C for 22.5 hours. The DMF was stripped and the residue vacuum dried. The residue was then taken up in 6 mL $H_2O$ and acidified to pH 1 using 26 drops of conc. HCl. The resultant solution was then applied to a 2.9 cm I.D. X 7 cm long column of AG 50W-X8 $NH_4$ form and eluted with 150 mL of water. The aqueous fractions were discarded, and the product was eluted with a step-gradient of 350 mL each of 0.5% and 0.7% ammonium hydroxide. The eluate was stripped to solvent dryness, and the residue placed under argon and left in the freezer overnight. The reddish residue was taken up with 8:2 mL methanol:$H_2O$, and applied to a 50 cm long X 3.8 cm I.D. column of LH-20. Elution with methanol gave 359.8 mg of 5-(1-aza-5,10-dioxa-13-aminotridecyl)-2′-deoxyuridine as shown in Figure 3 as a yellow oil upon lyophilization over 3 days; 32.7% yield.

## EXAMPLE 6

Preparation of 5-(1 aza-5,10-dioxa-13-aminotridecyl)-2′-deoxyuridine-5′-monophosphate:

To a suspension of 32.6 mg of 5-(1-aza-5,10-dioxa-13-aminotridecyl)-2′-deoxyuridine in 1.0 mL triethylphosphate, prepared according to Example 5, was added 21 microliters of P(O)Cl₃. The reaction mixture was stirred at 0° C for 2 hours. Then, 21 microliters more P(O)Cl₃ was added, and stirring was continued in a 4° C cold room for 21 hours longer. The nucleotide was precipitated by addition of 24 mL of ether. The precipitate was isolated by centrifugation, and the solids were taken up into 10 mL of water. The solution was placed in a 70° C water bath for 45 min. The solvents were stripped off and the residue was vacuum dried. The residue was taken up into 2 mL of water and the solution was applied to a 4 mL column of AG 50W-X8,$NH_4$ form. The column was washed with 32 mL of water, then the product was eluted using 30 mL of 1% ammonium hydroxide. After solvent removal, the residue was chromatographed on LH-20 to give 3.8 mg, 10% of 5-(1-aza-5,10-dioxa-13-amino-tridecyl)-2′-deoxyuridine-5′-monophosphate.

## EXAMPLE 7

Preparation of 5-(1-aza-5,10-dioxa-13-aminotridecyl)-2′-deoxyuridine-5′-triphosphate :

A 3.32 mg .sample of 5-(1-aza-5,10-dioxa-13-aminotridecyl)-2′-deoxyuridine-5′-monophosphate, prepared as described in Example 6, was suspended into 4 mL of methanol and 15 mL of NBu₃ was added.

The suspension was refluxed until a clear solution was attained. The methanol was stripped off and the residue was thoroughly vacuum dried and blanketed with argon. The residue was partially dissolved into 4:2 mL dioxane:DMF and 9 microliters of NBu$_3$ followed by the addition of 8 microliters of (PhO)$_2$POCl. The reaction mixture was stirred at room temperature for 4 h. The solvent was removed by rotary evaporation to give a volume of approximately 1 mL. The nucleotide was then precipitated using 100 mL Et$_2$O. The suspension was cooled in ice for 1 h and the resultant precipitate was isolated by centrifugation. The solid was taken up with dioxane and the dioxane was removed via rotary evaporation and vacuum drying. The residue was taken up into 4 mL of pyridine, and 12 mg of (NBu$_3$)$_{1.5}$H$_4$P$_2$O$_7$ [Sigma, St Louis, MO] was added. The mixture was stirred overnight at room temperature The crude pyridinium salt was precipitated with ether and the supernatant was decanted. The residue was vacuum dried and taken up into 10 mL, pH 3, aqueous HCl, and stirred at room temperature for 3 h. The solvent was stripped off and the reaction was vacuum dried, placed under an argon atmosphere, and kept in the freezer until it could be HPLC-chromatographed. Under the standard HPLC protocol conditions described in Example 3, the product, 5-(1-aza-5,10-dioxa-13-aminotridecyl)-2'-deoxyuridine-5'-triphosphate, as shown in Figure 4, had a retention time of 11.5 min.


## EXAMPLE 8


The nucleoside triphosphate of Example 3, 5-(1-aza-7-aminoheptyl)-2'-deoxyuridine-5'-triphosphate, was further derivatized with a reporter group comprising a chemiluminescent acridine derivative.


Preparation of chemiluminescent acridine derivatives:

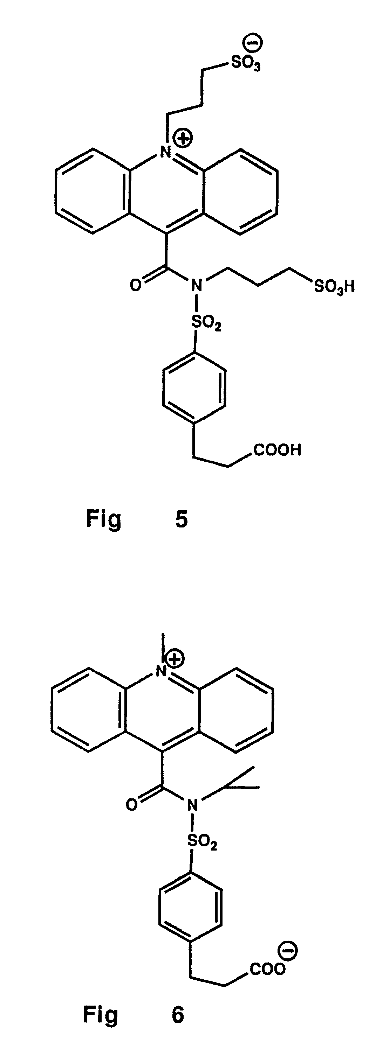

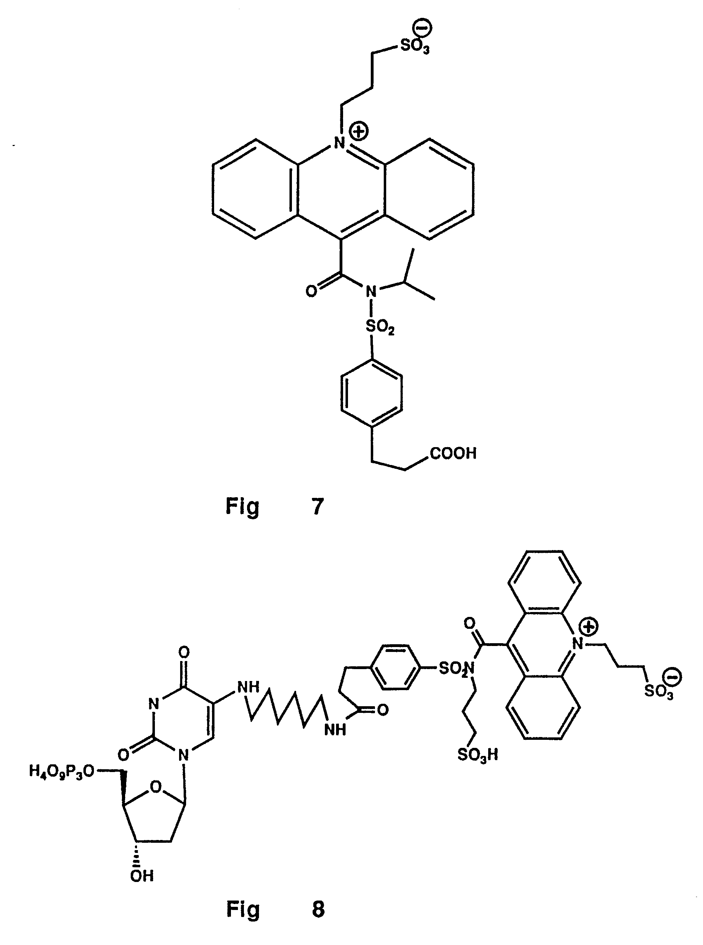

Methods for synthesis of chemiluminescent acridine derivatives are disclosed in a co-assigned and co-pending patent application U.S. Serial No. 921,979, filed October 22, 1986, which is incorporated herein by reference. The 3-(4-[N-(3-sulfopropyl)-N-[N'-(3-sulfopropyl)acridin-9-ylcarbonyl]sulfonamido]phenyl)propionic acid as shown in Figure 5, known hereinafter as SSC acridine, was synthesized as described hereinafter. The N-isopropyl, N'-methyl (MIC acridine) and the N-isopropyl, N'-sulfopropyl (SIC acridine) derivatives as shown in Figure 6 and Figure 7, respectively, were synthesized in a similar fashion.

The SSC acridine derivative was synthesized by the following method. To a solution of 1773 mg of 9-acridine-carboxylic acid chloride in 10 mL of dry tetrahydrofuran (THF) was added 320 mg of 50% NaH in mineral oil at room temperature. After the slight amount of foaming subsided, 1719 mg of ethyl 3-(4-sulfonamidophenyl)-propionate was added all at once. A large amount of gas was evolved, and the mixture turned brownish in color. The reaction mixture was then placed in a 55°C oil bath for 22 hours. The reaction was quenched by the addition of 4 mL of water. This turned the green suspension to a yellow-brown solution. The solvents were stripped off, and the residue taken up into MeOH. After centrifugation to remove the NaCl, the solvents were removed. The residue was taken up into 9:1 (v:v) CHCl$_3$:MeOH as much as possible and the solution was applied to a flash chromatography column made up in the same solvent system. Still, W., et al ., J. Org. Chem. , 43:2923 (1978). Collection of the second colored band afforded 2109.2 mg of ethyl 3-(4-[N-[N'-(3-sulfopropyl)acridin-9-ylcarbonyl]sulfonamido]phenyl)propionate, 69% yield. A 925 mg sample of the sulfonamide was thoroughly dried under vacuum overnight and 6 mL of propanesultone (Aldrich, Milwaukee, Wisconsin) was added The resultant suspension was heated in a 140°C oil bath for 4 hours. TLC analysis (9:1 CHCl$_3$:MeOH, silica gel) after this time showed that no starting material remained. 100 mL of benzene was then added. The resultant suspension was stirred until no more product was seen to precipitate. The benzene was decanted off and the crude precipitate was once again washed with benzene.

The crude material was taken up into 2:1 MeOH:H$_2$O and applied to a 3.5 cm ID x 50 cm long column of LH-20. Elution with methanol and collection of the large, yellow-green fluorescent fraction gave 1846 mg of a very thick gum. The entire sample of ethyl 3-(4-[N-(3-sulfopropyl)-N-[N'-(3-sulfopropyl)acridin-9-ylcarbonyl]sulfona mido]phenyl)propionate was taken up into 60 mL of 1 M HCl, and the yellow solution was refluxed for 4.5 hours. TLC analysis after this time (1:1 MeOH:CHCl$_3$) showed that no starting material remained. After solvent removal, elution of the crude material on LH-20 (3.5 cm ID x 50 cm) using methanol afforded 1066.8 mg of 3-(4-[N-(3-sulfopropyl)-N-[N'-(3-sulfopropyl)acridin-9-ylcarbonyl]sulfonamido]phenyl)-propionic acid. HPLC analysis under standard gradient protocol conditions, according to Example 3, showed the SSC acridine to elute at 11.04 minutes.

The MIC acridine and SIC acridine derivatives were synthesized in a similar fashion. MIC acridine was prepared from the reaction of N methyl-9-acridine carboxylic acid chloride, itself prepared from 9-acridine carboxylic acid and methyl trifluoromethane sulfonate, with ethyl 3-(4-phenylsulfonylisopropylamide) propionate. SIC acridine was prepared from the reaction of 9-acridine carboxylic acid chloride with ethyl 3-(4-phenylsulfonylisopropylamido) propionate, followed by reaction with propanesultone.

Preparation of dUTP acridine Adducts :

The nucleoside triphosphate of Example 3, 5-(1-aza-7-aminoheptyl)-2'-deoxyuridine-5'-triphosphate, was further derivatized by coupling it with SSC acridine as described hereinafter. In addition, the nucleoside triphosphate of Example 7 was coupled with SSC acridine. The coupling procedure was also used to couple the nucleoside triphosphates of Example 3 and Example 7 with the MIC acridine derivative as well as the SIC acridine derivative.

The reporter group-labeled nucleoside triphosphate 5-(1-aza-7-[3-(4-[N-(3-sulfopropyl)-N-[N'-(3-sulfopropyl)acridin-9-ylcarbonyl]sulfonamido]phenyl)propionamido]heptyl)-2'-deoxyuridine-5'-triphosphate (also referred to as the 5-(1-aza-7-aminoheptyl)-2'-dUTP-SSC acridine adduct), as shown in Figure 8, was synthesized by the following method. To a solution of 5.0 mg of 5-(1-aza-7-aminoheptyl)-2'-deoxyuridine-5'-triphosphate and 30.6 mg of SSC-acridine in 10 mL of DMF was added 7 mg of dicyclohexylcarbodiimide and 4 mg of N-hydroxysuccinimide. The reaction mixture was stirred at room temperature for 3 hours. TLC analysis (50:11:30 n BUOH:AcOH:$H_2O$) after this time showed that no starting triphosphate remained. After solvent removal, the residue was eluted down an LH-20 column using methanol. The fractions which remained at the origin on TLC were pooled and the material of the pooled fractions were purified by HPLC. Under the standard gradient protocol conditions, the 5-(1-aza-7-amino-heptyl)-2'-dUTP-SSC acridine adduct had a retention time of 10.4 minutes.

EXAMPLE 9

Preparation of a DNA oligonucleotide Incorporating 5-(1-aza-7-aminoheptyl)-2'-dUTP-SCC acridine adduct:

The 5-(1-aza-7-aminoheptyl)-2'-dUTP-SSC acridine adduct of Example 8 was incorporated into a DNA chain by the Klenow fragment of DNA polymerase as follows. A solution of 250 ng M13mp18 DNA (New England Biolabs, Beverly, MA), 2.5 ng M13 primer (New England Biolabs, #1202), and 1 microliter 10-X buffer (300 mM NaCl, 100 mM bisTris, 100 mM $MgCl_2$, pH 6.5) in 9 microliters water was boiled for 3 minutes; hybrid formation was initiated in a 65° C bath for 1 hour. After the solution was chilled to 0° C, 500 pmol each of dGTP and dCTP (Pharmacia, Piscataway, NJ) were added, followed by 500 pmol of the 5-(1-aza-7-aminoheptyl)-2'-dUTP-SSC acridine adduct and 12 pmol of alpha-P-32 ATP (Amersham, Arlington Heights, IL). Enzymatic incorporation was initiated by addition of 5 U of Klenow fragment. The reaction was allowed to proceed at 15° C for 2 hours and was quenched by the addition of 1 microliter of 0.5 M EDTA. The degree of incorporation was checked by descending paper chromatography on DEAE cellulose, developed with 300 mM ammonium formate. Autoradiography of the paper showed definite incorporation of the nucleotide into the DNA, as there was a great deal of radioactivity at the origin thereby demonstrating that the DNA polymerase accepted the oligonucleotide triphosphate as a bona fide substrate.

Example 10

Use of a Reporter Group Labeled Oligonucleotide as a Nucleic Acid Probe:

To a solution of 1 ng double-stranded M13mp18 DNA in 200 microliters of 50 mM $NaH_2PO_4$, pH 6.8/300 mM NaCl/0.1% sodium dodecylsulfate, was added 50 ng of the Klenow extended, labeled oligonucleotide, described in Example 9, dissolved in 100 microliters hybridization buffer. The solution was boiled for 5 min, then hybridized at 65° C overnight. The solution was then applied to a 17.5 cm long X 5 mm I.D. column of CL-4B (Pharmacia, Piscataway, NJ), equilibrated in hybridization buffer. Elution of the column with 850 microliters buffer and collection of the eluate gave the hybrid of interest. This material was radioactive and chemiluminescent positive, affirming hybrid formation.

The foregoing illustrative examples relate to the preparation and isolation of 5-position modified nucleosides and nucleotides and their use in the synthesis of and use of oligonucleotides. While the present invention has been described in terms of specific methods and compositions, it is understood that variations and modifications will occur to those skilled in the art upon consideration of the present invention. Accordingly it is intended in the appended claims to cover all such equivalent variations which come within the scope of the invention as claimed.

## Claims

1. A method for preparing a uracil nucleoside derivative of the formula:

S-U-NH-R

wherein:

S is a member selected from the group consisting of furanoses, pyranoses, cyclopentane derivatives, cyclohexane derivatives and straight or branched chain alkane derivatives of from 2 to 6 carbon atoms;

U is uracil; and

R is of the formula:

$-(CH_2)_u[(CH_2)_vO(CH_2)_w]_x[(CH_2)_vO(CH_2)_x]_y(CH_2)_z$-T

wherein:

u is 1 to 20,

v is 1 to 5,

w is 1 to 5,

x is 0 to 1,

y is 0 to 5,

z is 0 to 20; and

the sum of u, v, w, x, y, and z is 5 to 20; T is $-CH_2A$, $-CH-[CH_2)_nA]_2$, or $-C-[(CH_2)_nA]_3$;

n is 1 to 4; and

A is $NH_2$, SH, or OH;

comprising the steps of:

reacting a 5-halo-uracil nucleoside with a bifunctional amine of the formula:

$H_2N-(CH_2)_u[(CH_2)_vO(CH_2)_w]_x[(CH_2)_vO(CH_2)_w]_y(CH_2)_z$-T

wherein:

u is 1 to 20,

v is 1 to 5,

w is 1 to 5,

x is 0 to 1,

y is 0 to 5,

z is 0 to 20; and

the sum of u, v, w, x, y, and z is 5 to 20;

T is $-CH_2A$, $-CH-[(CH_2)_nA]_2$, or $-C-[(CH_2)_nA]_3$;

n is 1 to 4; and

A is $NH_2$, SH, or OH;

whereby a reaction mixture is formed containing a 5-aza uracil nucleoside derivative; and

isolating from said reaction mixture said 5-aza uracil nucleoside derivative.

2. The method according to claim 1, wherein S-U is selected from the group consisting of uridine, 2′-deoxyuridine, 2′,3′-dideoxyuridine, 2′-deoxyarabinosyluracil, and arabinosyluracil.

3. The method according to claim 1, wherein R is selected from group consisting of:

$-(CH_2)_3$-0-$(CH_2)_4$-0-$(CH_2)_3$-$NH_2$, and

$-(CH_2)_6 NH_2$; and

wherein said 5-halo-uracil nucleoside is a member selected from the group consisting of:

5-iodo-uracil nucleoside, 5-bromo-uracil nucleoside and 5-chloro-uracil nucleoside; and

wherein said bifunctional amine is a diamine selected from the group consisting of:

$NH_2(CH_2)_3$-0-$CH_2)_4$-0-$(CH_2)_3$-$NH_2$,

and

$NH_2(CH_2)_6 NH_2$.

12

4. A method for preparing a uracil nucleotide derivative of the formula:

P-S-U-NH-R

wherein:

P is a member selected from the group consisting of a monophosphate, diphosphate, and triphosphate;

S is a member selected from the group consisting of furanoses, pyranoses, cyclopentane derivatives, cyclohexane derivatives and straight or branched chain alkane derivatives of from 2 to 6 carbon atoms;

U is uracil; and

R is of the formula:

$-(CH_2)_u[(CH_2)_vO(CH_2)_w]_x[(CH_2)_vO(CH_2)_w]_y(CH_2)_z$-T-B

wherein:

u is 1 to 20,

v is 1 to 5,

w is 1 to 5,

x is 0 to 1,

y is 0 to 5,

z is 0 to 20; and

the sum of u, v, w, x, y, and z is 5 to 20;

T is $-CH_2A$,$-CH-[(CH_2)_nA]_2$, or $-C-[(CH_2)_nA]_3$; n is 1 to 4;

A is NH, S, or O; and

B is a reporter group;

comprising the steps of:

reacting a 5-halo-uracil nucleoside with a bifunctional amine of the formula:

$H_2N-(CH_2)_u[(CH_2)_vO(CH_2)_w]_x[(CH_2)_vO(CH_2)_w]_y(CH_2)_z$-T

wherein:

u is 1 to 20,

v is 1 to 5,

w is 1 to 5,

x is 0 to 1,

y is 0 to 5,

z is 0 to 20; and

the sum of u, v, w, x, y, and z is 5 to 20;

T is $-CH_2A$,$-CH-[(CH_2)_nA]_2$, or$-C-[(CH_2)_nA]_3$;

n is 1 to 4;

A is $NH_2$, SH, or OH;

whereby a first reaction mixture is formed containing 5-aza uracil nucleoside derivative with a reactive terminus;

phosphorylating said 5 aza uracil nucleoside derivative to form a 5-aza uracil nucleotide derivative;

condensing the free amino, sulfhydryl or hydroxyl group of said reactive terminus of said 5-aza uracil nucleotide derivative with a reporter group, whereby a second reaction mixture is formed containing a reporter group labeled 5-aza uracil nucleotide derivative; and

isolating from said second reaction mixture said reporter group labeled 5-aza uracil nucleotide derivative.

5. A uracil nucleoside derivative of the formula:

P-S-U-NH-R

wherein:

P is a member selected from the group consisting of hydroxyl, monophosphate, diphosphate and triphosphate;

S is a member selected from the group consisting of furanoses, pyranoses, cyclopentane derivatives, cyclohexane derivatives and straight or branched chain alkane derivatives of from 2 to 6 carbon atoms;

U is uracil; and

R is of the formula:

$-(CH_2)_u[(CH_2)_vO(CH_2)_w]_x[(CH_2)_vO(CH_2)_w]_y(CH_2)_z$-T-B

wherein:

u is 1 to 20,

v is 1 to 5,

w is 1 to 5,

x is 0 to 1,

y is 0 to 5,

z is 0 to 20; and

the sum of u, v, w, x, y, and z is 5 to 20;

T is $-CH_2A,-CH-[(CH_2)_nA]_2$, or $-C-[(CH_2)_nA]_3$;

n is 1 to 4;

A is NH, S, or O; and

B is H or a reporter group.

6. The uracil nucleoside derivative of claim 5, wherein P is triphosphate, and wherein B is a reporter group selected from the group consisting of fluorescent compounds, chemiluminescent compounds, non-radioactive isotopes, metal chelators, biotin, antigens, enzymes and liposomes.

7. The uracil nucleoside derivative of claim 5, wherein S-U is selected from the group consisting of uridine, 2′-deoxyuridine, 2′, 3′-dideoxyuridine, 2′-deoxyarabinosyluracil, and arabinosyluracil; and wherein R is selected from the group consisting of:

$-(CH_2)_3-0-(CH_2)_4-0-(CH_2)_3-NH-B$, and

$-(CH_2)_6NH-B$.

8. The uracil nucleoside derivative of claim 5, wherein P-S-U-NH-R is a member selected from the group consisting of:

5-(1-aza-7-aminoheptyl)-2′-deoxyuridine, 5-(1-aza-7-aminoheptyl)-2′-deoxyuridine-5′-triphosphate, 5-(1-aza-7-fluoresceinthioureaheptyl)-2′-deoxyuridine-5′-triphosphate, 5-(1-aza-5,10 dioxa-13-aminotridecyl)-2′-deoxyuridine, 5-(1-aza-5,10-dioxa-13-aminotridecy)-2′-deoxyuridine-5′-triphosphate, 5-(1-aza-7 aminoheptyl)-2′-dUTP-SSC acridine adduct and 5-(1-aza-5,10-dioxa-13-aminotridecyl)-2′-dUTP-SSC acridine adduct.

9. An oligonucleotide according to claim 5 comprising a uracil nucleoside derivative wherein said uracil nucleoside derivative is a member selected from the group consisting of:

5-(1-aza-7-aminoheptyl)-2′-deoxyuridine-5′-triphosphate, 5-(1-aza-7-fluoresceinthioureaheptyl)-2′-deoxyuridine-5′-triphosphate, 5-(1-aza-5,10-dioxa-13-aminotridecyl)-2′-deoxyuridine-5′-triphosphate, 5-(1-aza-7-aminoheptyl)-2′-dUTP-SSC acridine adduct and 5-(1-aza-5,10-dioxa-13-aminotridecyl)-2′-dUTP-SSC acridine adduct.

10. In an automated method for preparing reporter group-labeled oligonucleotides comprising the steps of:

(a) suitably protecting four substrates, dA, dG, dC and dT,

(b) synthesizing the 5′-dimethoxytrityl-3′-beta-cyano-ethylphosphoramidite derivatives of said four substrates,

(c) adding the derivative of one of said four substrates to a solid phase bound DNA molecule by dissolving said derivative in an anhydrous solvent to form an adduct,

(d) washing said adduct with organic acid,

(e) adding another of the derivatives of said four substrates synthesized in step (b), whereby said oligonucleotides are formed, and

(f) labeling with a reporter group said oligonucleotides so formed, the improvement comprising using as one of said four substrates in step (a) the uracil nucleoside derivative of claim 5.

Fig 1

Fig 2

Fig        3

Fig        4

Fig    5

Fig    6

**Fig 7**

**Fig 8**